# EUROPEAN PATENT APPLICATION

(11) **EP 1 970 565 A1**
(43) Date of publication of application: **17.09.2008**
(21) Application number: 07118466.7
(22) Date of filing: 15.10.2007
(51) Int. Cl.: F04B 19/00, B01L 3/00, F16K 99/00

(54) **Pump unit and centrifugal microfluidic system having the same**

(30) Priority: 14.03.2007 KR 20070025148
(71) Applicant: Samsung Electronics Co., Ltd., Suwon-si, Gyeonggi-Do (KR)
(72) Inventor: Park, Jong-myeon Samsung Advanced Institute of Technology, Gyeonggi-do (KR); Huh, Nam Samsung Advanced Institute of Technology, Gyeonggi-do (KR); Lee, Jeong-gun Samsung Advanced Institute of Technology, Gyeonggi-do (KR); Hwang, Kyu-youn Samsung Advanced Institute of Technology, Gyeonggi-do (KR)
(74) Representative: Grünecker, Kinkeldey, Stockmair & Schwanhäusser Anwaltssozietät

(57) **Abstract**

Provided are a pump unit (30) and a centrifugal microfluidic system including the pump unit. The pump unit (30) includes a gas generating agent (32). The gas generating agent (32) includes a plurality of heating particles generating heat by absorbing energy and one of a sublimation material, azide, and azo compound mixed with the heating particles. When energy (L) is supplied to the gas generating agent, the gas generating agent (32) generates gas due to hear emitted from the heating particles to increase an air pressure around the gas generation agent so that the fluid (F) is moved by the increased air pressure.

## Description

### BACKGROUND OF THE INVENTION

### 1. Field of the Invention

The present invention relates to micro fluidics, and more particularly, to a pump unit pumping a fluid through a micro channel and a centrifugal microfluidic system including the pump unit.

### 2. Description of the Related Art

Microfluidic systems are used in microfluidic engineering fields for various purposes, such as separating or mixing fluids, performing biochemical reactions, and measuring the results of biochemical reactions. For example, a microfluidic system may include a substrate type device such as a lab-on-a-chip. For microfluidic processing, the substrate type device includes a micro channel forming a fluid passage and a chamber receiving and/or processing a fluid. The microfluidic system may further include a pump unit for generating a fluid stream through the micro channel.

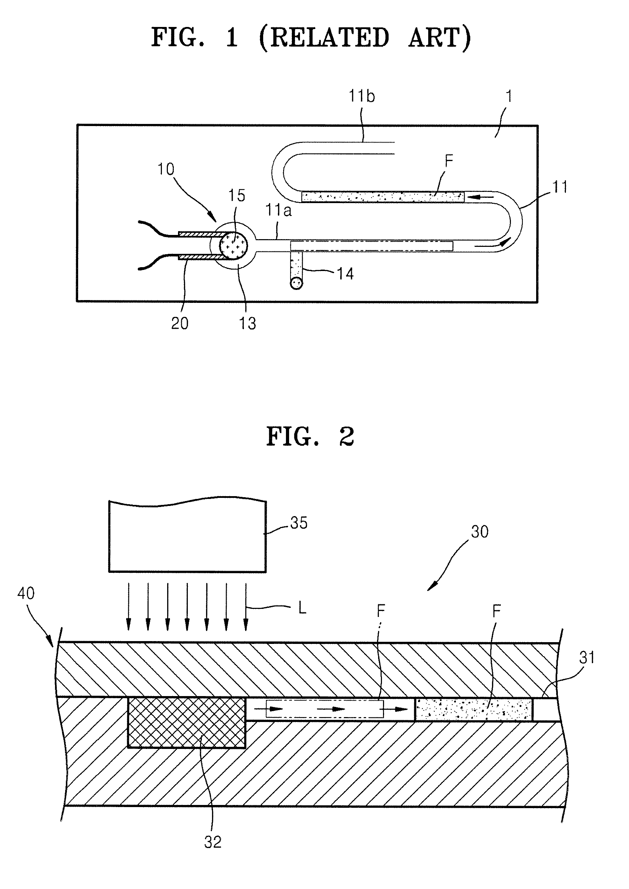

FIG. 1 illustrates a conventional pump unit 10 used for pumping a fluid through a micro channel. The pump unit is disclosed in US Application No. 10/946,818 (published as Application Publication No. 20050232817A), the disclosure of which is incorporated herein by reference.

Referring to FIG. 1, the pump unit 10 includes a main channel 11 formed in a substrate 1, a propellant chamber 13 formed at an end 11a of the main channel 11, and a fluid injection channel 14 formed close to the propellant chamber 13 and connected to the main channel 11. A propellant 15 is filled in the propellant chamber 13. The propellant 15 is a mixture of poly tetra fluoro ethylene (PTFE) known as Teflon® and azobis-isobutyronitrile (AIBN). When heated to a temperature of about 70°C or higher, the AIBN generates N₂ gas.

The pump unit 10 further includes a heater 20 to heat the propellant 15. The heater 20 generates heat using electricity and is formed in the substrate 1 or outside the substrate 1 to be in contact with the substrate 1. A fluid (F) is injected into the main channel 11 through the fluid injection channel 14, and heat is generated from the heater 20 using electricity to heat the propellant 15 and generate N₂ gas from the propellant 15. Then, the fluid (F) is moved from the end 11a of the main channel 11 toward the other end 11b by a pressure applied to the fluid (F) by the N₂ gas. The movement of the fluid (F) is illustrated by an imaginary dash-point line, a solid line, and arrows therebetween. However, it is difficult to reduce the size of the substrate 1 and integrate the substrate 1 since the heater 20 should be formed inside or in contact with the substrate 1.

Meanwhile, in the field of microfluidics, a centrifugal microfluidic system is well known, which includes a compact disk shaped substrate (CD substrate). A fluid can be pumped outward using a centrifugal force by rotating the CD substrate. The centrifugal microfluidic system can have a highly integrated structure. However, in the centrifugal microfluidic system, a fluid can be pumped only in one way from the center to the periphery. Furthermore, it is difficult to apply the pump unit 10 illustrated in FIG. 1 to the centrifugal microfluidic system for pumping a fluid from the periphery to the center of the centrifugal microfluidic system since the pump unit 10 includes the heater 20 requiring electricity. Therefore, it is difficult to design a centrifugal microfluidic system that can be used for performing a microfluidic treatment process including a complex biochemical reaction and a detection process for the reaction using a single CD substrate.

### SUMMARY OF THE INVENTION

The present invention provides a pump unit applying a pumping force to a fluid by supplying energy to a gas generating agent in a non-contact manner, and a centrifugal microfluidic system including the pump unit.

According to an aspect of the present invention, there is provided a pump unit including a chamber connected to and fluid communicated to the first channel, the chamber being disposed at a upstream side of the fluid which is to be moved toward a downstream side; and a gas generating agent contained in the chamber, wherein the gas generating agent includes a plurality of heating particles or a mixture of the plurality of heating particles and one of a sublimation material, azide, and azo compound; and wherein the gas generating agent increases air pressure when an energy is applied thereto, thereby moving the fluid toward the downstream direction. The pump unit may further include an external energy source. The chamber is connected to the first channel through a second channel.

According to another aspect of the present invention, there is provided a centrifugal microfluidic system including: a substrate including a channel constituting a fluid passage; and a pump unit moving a fluid in the first channel, wherein the pump unit includes a chamber connected to and fluid communicated to the first channel, the chamber being disposed at a upstream side of the fluid which is to be moved toward a downstream side; and a gas generating agent contained in the chamber, wherein the gas generating agent includes a plurality of heating particles or a mixture of the plurality of heating particles and one of a sublimation material, azide, and azo compound; and wherein the gas generating agent increases air pressure when an energy is applied thereto, thereby moving the fluid toward the downstream direction. The system may further include a driving unit rotating the substrate. The system may further include an external energy source. The chamber is connected to the first channel through a second channel.

The gas generating material may be a mixture of the plurality of the heating particles and a sublimation material. The sublimation material may be one selected from the group consisting of naphthalene, dry ice, iodine, camphor, and paradichlorobenzene. The sublimation material may be present in the form of powder.

The azide may be one selected from the group consisting of an inorganic azide, alkyl azide, and aryl azide. The azide may be present in the form of powder.

The inorganic azide may be NaN₃.

The azo compound may be one selected from the group consisting of AIBN (Azobisisobutyronitrile), DVN (2,2'-Azobis-(4-methoxy-2,4-dunethylvaleronitrile)), AMBN (2,2'-Azobis-(2-methylbutyronitrile)), CHN (1,1'-Azobis-(4-cyclohexanecarbonitrile)), ACCN (1,1'-Azobis-(cyclohexanecarbonitrile)), ABAH (2,2'-Azobis-(2-methylbutyronitrile)), and ACVA (1,1'-Azobis-(cyclohexanecarbonitrile)). The azo compound may be present in the form of powder.

The external energy source may emit electromagnetic waves towards the gas generating agent.

The electromagnetic waves may be in the form of a laser beam, and the external energy source may include a laser source generating a laser beam.

The laser source may generate a continuous-wave laser beam having an output power of at least 10 mW.

The external energy source may emit electromagnetic waves towards the gas generating agent while scanning from an end of the gas generating agent to the other end of the gas generating agent.

The pump unit further may include a valve closing the second valve. The valve may be located in the second channel (pump channel) between the fluid and the gas generating agent so as to prevent the fluid from flowing to the gas generating agent, wherein the valve opens the pump channel when the gas generating agent increases air pressure in the pump channel by an application of energy, thereby moving the fluid toward the downstream direction.

The valve may include a valve filler filled in at least part of the pump channel, the valve filler including a phase transition material, wherein when energy is supplied to the valve filler, a viscosity of the valve filler reduces, thereby opening the pump channel..

The phase transition material may be one selected from the group consisting of wax, gel, and a thermoplastic resin.

The valve filler may further include a plurality of heating particles dispersed into the phase transition material, the heating particles generating heat by absorbing energy.

The heating particles may be ferromagnetic particles.

The heating particles may be metal oxide particles.

The metal oxide particles may be Al₂O₃, TiO₂, Ta₂O₃, Fe₂O₃, Fe₃O₄, or HfO₂.

The gas generating agent and the valve may be supplied with energy by a single external energy source.

### BRIEF DESCRIPTION OF THE DRAWINGS

The above and other features and advantages of the present invention will become more apparent by describing in detail exemplary embodiments thereof with reference to the attached drawings in which:

FIG. 1 is a plan view illustrating a conventional pump unit;

FIG. 2 is a view illustrating a pump unit according to an embodiment of the present invention;

FIG. 3 is a perspective view illustrating a centrifugal microfluidic system including a pump unit according to an embodiment of the present invention;

FIG. 4 is a sectional view taken along line IV-IV of FIG. 3, according to an embodiment of the present invention;

FIG. 5A is a photographic image illustrating results of a pumping performance test performed on a pump unit including a mixture of iron oxide powder and naphthalene powder as a gas generating agent;

FIG. 5B is a photographic image illustrating results of a pumping performance test performed on a pump unit including iron oxide powder as a heating agent;

FIG. 5C is a photographic image illustrating results of a pumping performance test performed on a pump unit including naphthalene powder as a gas generating agent; and

FIGS. 6 and 7 plan views illustrating pump units according to another embodiment of the present invention.

### DETAILED DESCRIPTION OF THE INVENTION

A pump unit and a centrifugal microfluidic system including the pump unit will now be described more fully with reference to the accompanying drawings, in which exemplary embodiments of the invention are shown.

FIG. 2 is a view illustrating a pump unit 30 according to an embodiment of the present invention.

Referring to FIG. 2, the pump unit 30 includes a gas generating agent 32 disposed close to a fluid (F). The fluid (F) is in a channel 31 and may be stationary. An energy source 35 supplies energy to the gas generating agent 32. The gas generating agent 32 and the fluid (F) can be contained in a fluid treatment substrate 40 such as a lab-on-a-chip. The external energy source 35 supplies energy to the gas generating agent 32. In an embodiment, it does not make contact with the substrate 40 or the gas generating agent 32. The external energy source 35 may supply energy to the gas generating agent 32 by radiating electromagnetic waves. For example, the external energy source 35 may include a laser source, such as a laser diode (LD), emitting a laser beam (L). The substrate 40 may be transparent so that a laser beam (L) emitted from the external energy source 35 can be incident onto the gas generating agent 32 with less energy loss.

The gas generating agent 32 may be a mixture of heating particles and a sublimation material. The sublimation material my be present in the form of powder. The heating particles generate heat when they absorb energy. The sublimation material powder changes directly into gas from solid without an intermediate liquid phase when being heated under atmospheric pressure. The sublimation material powder may be formed of a material selected from the group consisting of naphthalene, dry ice, iodine, camphor, and paradichlorobenzene.

The heating particles have a diameter of several tens to several hundreds of nanometers. When energy is supplied to the heating particles, for example, by laser radiation, the heating particles increase in temperature rapidly and emit heat. The heating particles may be ferromagnetic metal oxide particles. For example, the heating particles may include Al₂O₃, TiO₂, Ta₂O₃, Fe₂O₃, Fe₃O₄ or HfO₂.

The sublimation material varies in vapor pressure with respect to temperature. When the external energy source 35 emits a laser beam (L) onto the gas generating agent 32, the heating particles (not shown) included in the gas generating agent 32 are rapidly heated by the laser beam (L), and thus the sublimation material changes into gas, increasing its volume and air pressure. As a result, an increased pressure is applied to fluid (F) to be moved into a direction opposite from the location of the gas generating agent 32. For example, when the fluid (F) is positioned at is stationary or starting point shown by an imaginary line, the pressure between the gas generating agent 32 and the fluid (F) at the stationary position is increased, and the fluid (F) is moved away from the gas generating agent 32 by the increased pressure to another position shown by a solid line. A continuous-wave laser beam having a power of 10 mW or larger can heat the gas generating agent 32. For example, rapid fluid pumping can be provided by a continuous-wave laser beam having a power of 1.5 W to 2.0 W.

The gas generating agent 32 of the pump unit 30 may be a mixture of heating particles and azide or azo compound powder. The azide or azo compound powder decomposes and generates N₂ gas when being heated. When the external energy source 35 irradiates a laser beam (L) onto the gas generating agent 32, the heating particles included in the gas generating agent 32 are rapidly heated by the laser beam (L), and thus the azide or azo compound powder composes to generate N₂ gas, increasing its volume. As a result, an increased pressure is applied to fluid (F) to be moved into a direction opposite from the location of the gas generating agent 32. For example, when the fluid (F) is positioned at is stationary or starting point shown by an imaginary line, the pressure between the gas generating agent 32 and the fluid (F) at the stationary position is increased, and the fluid (F) is moved away from the gas generating agent 32 by the increased pressure to the position shown by the solid line.

The azide may be one selected from the group consisting of an inorganic azide, alkyl azide, and aryl azide, and the inorganic azide may be NaN₃.

The azo compound may be one selected from the group consisting of AIBN (Azobisisobutyronitrile), ADVN (2,2'-Azobis-(4-methoxy-2,4-dimethylvaleronitrile)), AMBN (2,2'-Azobis-(2-methylbutyronitrile)), ACHN (1,1'-Azobis-(4-cyclohexanecarbonitrile)), ACCN (1,1'-Azobis-(cyclohexanecarbonitrile)), ABAH (2,2'-Azobis-(2-methylbutyronitrile)), and ACVA (1,1'-Azobis-(cyclohexanecarbonitrile)).

The pump unit 30 may include a heating agent 33 that includes heating particles but does not include sublimation material powder, azide powder, or azo compound powder. When a laser beam (L) is irradiated onto the heating agent 33, the heating particles generate heat rapidly, and thus air between the heating agent 33 and the fluid (F) at the original position shown by the imaginary line is increased in temperature and pressure. Therefore, the fluid (F) is pushed by the increased pressure to the position shown by the solid line. The heating agent 33 including only the heating particles is less effective as compared with the gas generating agent 32 in terms of pumping performance.

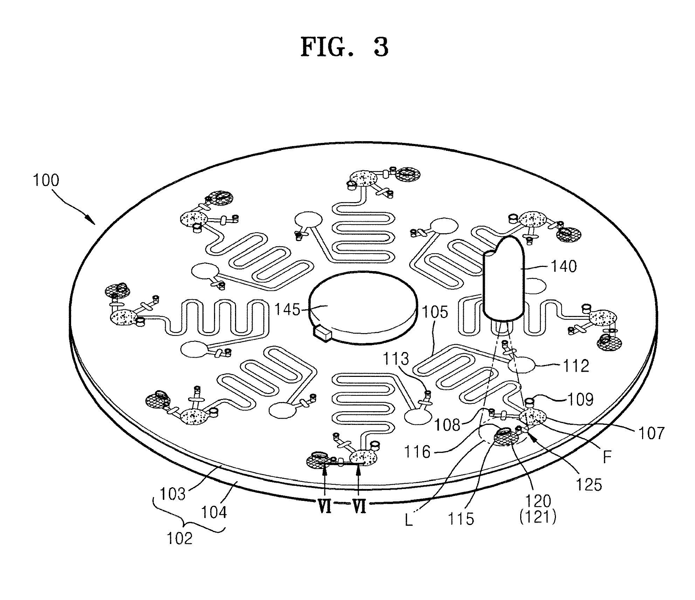

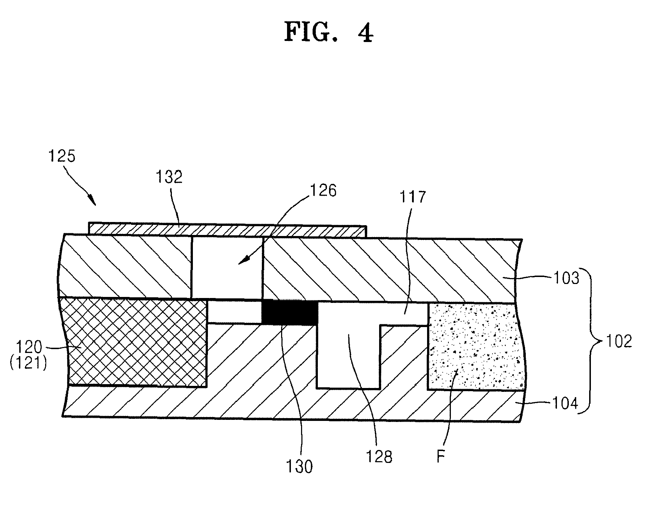

FIG. 3 is a perspective view illustrating a centrifugal microfluidic system 100 including a pump unit according to an embodiment of the present invention, and FIG. 4 is a sectional view taken along line IV-IV of FIG. 3, according to an embodiment of the present invention.

Referring to FIGS. 3 and 4, the centrifugal microfluidic system 100 includes a compact disk (CD) shaped substrate 102, a spindle motor 145 rotating the substrate 102, and an external energy source 140 emitting a laser beam (L) onto the substrate 102. The substrate 102 includes upper and lower plates 103 and 104 bonded together. The upper and lower plates 103 and 104 can be bonded together by double sided adhesive tape (not shown) or ultrasonic welding. At least the upper plate 103 is transparent to reduce energy loss of a laser beam (L).

The substrate 102 further includes main channels 105 forming fluid passages, first and second fluid chambers 107 and 112 connected to either end of the main channels 105, accommodation chambers 115 formed adjacent to the first fluid chambers 107, and pump channels 117 connecting the first fluid chambers 107 and the accommodation chambers 115. The first fluid chambers 107 in which fluids (F) are contained are positioned farther from a rotation center of the substrate 102 (i.e., the spindle motor 145) than the second fluid chambers 112. Reference numeral 108 denotes fluid injection holes for injecting fluids into the first fluid chambers 107, reference numeral 109 denotes vent holes of the first fluid chambers 107, and reference numeral 113 denotes vent holes of the second fluid chambers 112.

A gas generating agent 120 or heating agent 121 are filled in the accommodation chamber 115. As explained with reference to FIG. 2, the gas generating agent 120 may be a mixture including heating particles and sublimation material powder, or the gas generating agent 120 may be a mixture including heating particles and azide or azo compound powder. The heating agent 121 includes heating particles but does not include sublimation material powder, azide powder, or azo compound powder. Reference numeral 116 denotes an injection hole for injecting the gas generating agent 120 or the heating agent 121 into the accommodation chamber 115.

The centrifugal microfluidic system 100 further includes valve units 125 in the pump channels 117 between the accommodation chambers 115 and the first fluid chambers 107. Each valve unit 125 can close the pump channel 117 to prevent the fluid (F) from moving from the first fluid chamber 107 to the accommodation chamber 115 in which the gas generating agent 120 or the heating agent 121 is filled. The valve unit 125 includes a valve filler 130 contained in the pump channel 117. The valve filler 130 includes a phase transition material and heating particles for generating heat upon application of energy. The phase transition material may be dispersed in the phase transition material. The energy may be applied by an external energy source. In an embodiment, a single energy source may be used to supply energy to the gas generating agent 120 (or heating agent 121) and the valve filler 130 at the same time.

The phase transition material may be wax, gel, or a thermoplastic resin. For example, the wax may be paraffin wax. The gel may be polyacrylamide, polyacrylates, polymethacrylates, or polyvinylamides. The thermoplastic resin may be COC (cyclic olefin copolymer), PMMA (polymethylmethacrylate), PC (polycarbonate), PS (polystyrene), POM (polyoxymethylene), PFA (perfluoralkoxy), PVC (polyvinylchloride), PP (polypropylene), PET (polyethylene terephthalate), PEEK (polyetheretherketone), PA (polyamide), PSU (polysulfone), or PVDF(polyvinylidene fluoride).

The heating particles included in the valve filler 130 may be the same material as those explained with respect to the gas generating agent 120. Thus, description of the heating particles of the valve filler 130 will be omitted. The heating particles of the valve filler 130 can be dispersed in a hydrophobic carrier oil. The valve filler 130 can be formed by mixing a molten phase transition material with a hydrophobic carrier oil containing heating particles dispersed therein.

In one embodiment, one of the upper and lower plates, for example the lower plate 104 may include a sunk area 128 for receiving a molten valve filler 130, and the upper plate 103 may include a valve filler injection hole 126 for injecting a molten valve filler 130 into the pump channel 117. The valve filler injection hole 126 is not aligned with the sunk area 128. When a molten valve filler 130 is injected into the pump channel 117 through the valve filler injection hole 126, some of the molten valve filler 130 flows into the sunk area 128 and the rest of the molten valve filler 130 may remain in the pump channel 117 between the valve filler injection hole 126 and the sunk area 128. Then, the valve filler 130 remaining in the pump channel 117 decreases its viscosity (e.g., solidification), thereby closing the pump channel 117. After a valve filler 130 is injected through the valve filler injection hole 126, the valve filler injection hole 126 can be sealed with a proper member such as tape 132 to prevent permeation of air. Other modifications and changes may be made to the valve unit 125. Such modifications and changes are described in a co-pending application Serial No. , disclosures of which are incorporated by reference.

The CD shaped substrate 102 is coupled to the spindle motor 145, and the spindle motor 145 is driven to rotate the substrate 102 at a high speed. Then, a fluid injected into the substrate 102 is moved in a radial direction of the substrate 102. That is, a fluid contained in the main channel 105 is moved from the center of the substrate 102 to the periphery.

A fluid (F) is injected into the first fluid chamber 107, and a gas generating agent 120 or a heating agent 121 is injected into the accommodation chamber 115. Next, the fluid injection hole 108, the vent hole 109, and the injection hole 116 are closed with tape (not shown) or the like. Thereafter, a laser beam (L) is irradiated on the gas generating agent 120 (or the heating agent 121) and the valve filler 130 filled in the pump channel 117 using the external energy source 140. Then, the valve filler 130 melts, and thus the pump channel 117 is opened. Meanwhile, heating particles included in the gas generating agent 120 or the heating agent 121 generate heat rapidly and increase the pressure of the pump channel 117. As a result of the increased pressure, the fluid (F) contained in the first fluid chamber 107 is moved away from the gas generating agent 120 or the heating agent 121 toward the center of the substrate 102 through the main channel 105. The fluid moves into the second chamber 112, where the fluid may be processed or reacted appropriately.

Even though Figs. 3 and 4 illustrate an exemplary configuration of centrifugal microfluidic system, it should be noted that the configuration of channels, chambers, holes and other components of the system may be modified depending on the particular uses or needs of the centrifugal microfluidic system. Such modification is within the knowledge and practice of one skilled in the art.

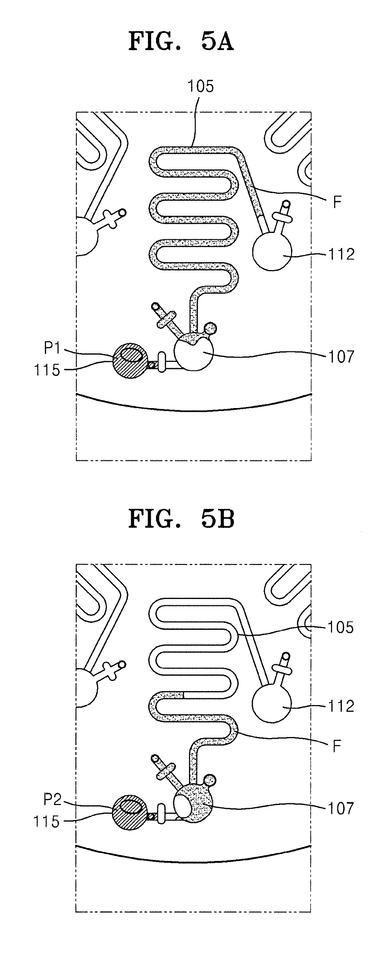

A pumping performance test was performed to compare the performance of the pump unit in the case of using the gas generating agent 120 with that in the case of the heating agent 121, and the test results are shown in FIGS. 5A through 5C. In the pumping performance test, a 1.5-W continuous-wave laser beam (L) was irradiated from the external energy source 140 on the gas generating agent 120, the heating agent 121, and the valve fill 130 for fifteen seconds.

In the case of FIG. 5A, a gas generating agent P1 was used. The gas generating agent P1 was a mixture including iron oxide powder as heating particles and naphthalene powder as sublimation material powder. Most of a fluid (F) contained in the first fluid chamber 107 was pumped to the second fluid chamber 112 through the main channel 105. That is, it can be understood that the gas generating agent P1 formed of heating particles and sublimation material powder provides good fluid pumping performance.

In the case of FIG. 5B, a heating agent P2 formed of only iron oxide powder was used. A fluid (F) contained in the first fluid chamber 107 was pumped to a middle part of the main channel 105. It can be understood that the heating agent P2 provides good fluid pumping performance to some degree although the heating agent P2 provides relatively lower pumping performance than the gas generating agent P1 (refer to FIG. 5A).

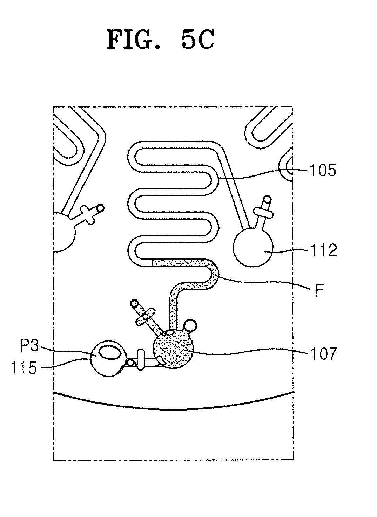

In the case of FIG. 5C, a gas generating agent P3 formed of only naphthalene powder is used. A fluid (F) contained in the first fluid chamber 107 was not well pumped. The reason for this is that it is difficult to heat a sublimation material such as naphthalene to a sufficiently high temperature for sublimation by supplying energy to the sublimation material by a non-contact type method such as using a laser beam (L). Therefore, the gas generating agent P3 formed of only a sublimation material without heating particles is not used in the pump unit 30 (refer to FIG. 2) or the centrifugal microfluidic system 100 (refer to FIG. 3) of the present invention.

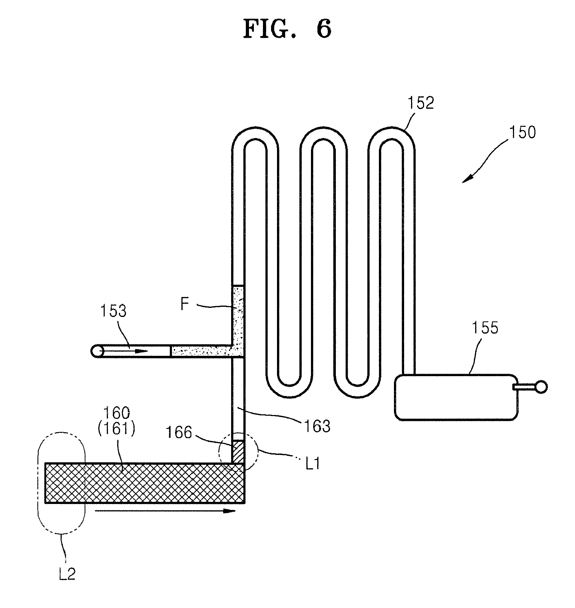

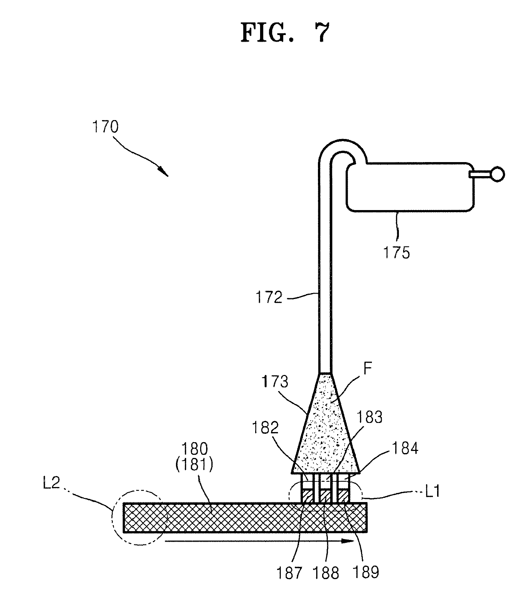

FIGS. 6 and 7 are plan views illustrating pump units according to another embodiments of the present invention.

Referring to FIG. 6, a pump unit 150 of the current embodiment includes a main channel 152 forming a fluid passage, a fluid inlet channel 153 connected to an end of the main channel 152, a gas generating agent 160 connected to the end of the main channel 152 through a pump channel 163, and a fluid chamber 155 connected to the other end of the main channel 152. The pump unit 150 further includes an external energy source (not shown) emitting a laser beam (L2) on the gas generating agent 160, and a valve closing and opening the pump channel 163.

The gas generating agent 160 may be a mixture of heating particles and a sublimation material, azide, or azo compound. The sublimation material, azide or azo compound may be used as powder. The gas generating agent 160 is the same material as the one explained above with respect to the gas generating agent 32 of the pump unit 30 illustrated in FIG. 2. Thus, detailed description of the gas generating agent 160 will be omitted. Alternatively, the pump unit 150 can include a heating agent 161 formed of only heating particles in replacement of the gas generating agent 160. The heating agent 161 is the same as the heating agent 33 of the pump unit 30 of FIG. 2, and thus description of the heating agent 161 will be omitted.

The value includes a valve filler 166 filled in the pump channel 163. The valve filler 166 and the valve are the same as those explained above with respect to the valve filler 130 and the valve 125 of the centrifugal microfluidic system 100 illustrated in FIGS. 3 and 4. Thus, detailed descriptions of the valve filler 166 and the valve including the valve filler 166 will be omitted. A laser beam (L1) can be irradiated on the valve filler 166 using the same external energy source (not shown) used to irradiate a laser beam (L2) on the gas generating agent 160, or using an additional external energy source.

A fluid (F) is injected into the main channel 152 through the fluid inlet channel 153, and a laser beam (L1) is irradiated on the valve filler 166 to open the pump channel 163. Next, a laser beam (L2) is irradiated on the gas generating agent 160. Then, the heating particles included in the gas generating agent 160 generate heat rapidly, and thus the sublimation material rapidly sublimates. As a result, the pressure of the pump channel 163 increases. Therefore, the fluid (F) filled in a portion of the main channel 152 is moved by the increased pressure away from the gas generating agent 160 toward the fluid chamber 155 through the main channel 152.

The fluid pumping performance of the pump unit 150 can be improved by increasing the amount of the gas generating agent 160. However, in this case, the external energy source should include more laser diodes (LDs) to simultaneously irradiate the gas generating agent 160 disposed in a relatively large area, and the external energy source will consume more power. Thus, the cost for operation will increase. This cost increase can be avoided by using an external energy source which can move along, while it irradiates onto the gas generating agent 160, from one end to the other end of the gas generating agent 160 which occupies a relatively large area, as shown by the arrow next to the gas generating agent 160 in FIG. 6. In this way, the fluid pumping performance of the pump unit 150 can be improved at low cost by increasing the amount of the gas generating agent 160 without increasing the number of LDs of the external energy source 35.

Referring to FIG. 7, a pump unit 170 of the current embodiment includes a main channel 172 forming a fluid passage, a first fluid chamber 173 connected to an end of the main channel 172, and a second fluid chamber 175 connected to the other end of the main channel 172. The pump unit 170 further includes a gas generating agent 180 connected to an end of the first fluid chamber 173 through pump channels 182, 183, and 184, an external energy source (not shown) emitting a laser beam (L2) on the gas generating agent 180, and valves closing and opening the pump channels 182, 183, and 184.

The first fluid chamber 173 has a triangular shape such that a fluid (F) contained in the first fluid chamber 173 can converge into the main channel 172. A side of the first fluid chamber 173 connected to the pump channels 182, 183, and 184 is wider than a side of the first fluid chamber 173 connected to the main channel 172. Since there are three pump channels 182, 183, and 184, a pumping force can be uniformly applied to the fluid (F) through the pump channels 182, 183, and 184. Meanwhile, the valves corresponding to the pump channels 182, 183, and 184 are also three in number. The valves include valve fillers 187, 188, and 189 filled in the pump channels 182, 183, and 184, respectively. The valves and the valve fillers 187, 188, and 189 are the same as those explained above with respect to the valve 125 and the valve fill 130 of the centrifugal microfluidic system 100 illustrated in FIGS. 3 and 4, and thus descriptions thereof will be omitted.

The gas generating agent 180 may be a mixture of heating particles and a sublimation material, azide, or azo compound. The sublimation material, azide or azo compound may be used in the form of powder. The gas generating agent 180 is the same as the gas generating agent 32 of the pump unit 30 illustrated in FIG. 2. Thus, detailed description of the gas generating agent 180 will be omitted. Alternatively, the pump unit 170 can include a heating agent 181 formed of only heating particles in replacement of the gas generating agent 180. The heating agent 181 is the same as the heating agent 33 of the pump unit 30 of FIG. 2, and thus description of the heating agent 181 will be omitted.

A laser beam (L1) can be irradiated on the valve fillers 187, 188, and 189 using an external energy source used to irradiate a laser beam (L2) on the gas generating agent 180, or using an additional external energy source.

A fluid (F) is injected into the first fluid chamber 173, and a laser beam (L1) is irradiated on the valve fillers 187, 188, and 189 to open the pump channels 182, 183, and 184. Next, a laser beam (L2) is irradiated on the gas generating agent 180. Then, the heating particles included in the gas generating agent 180 generate heat rapidly, and thus the sublimation material powder rapidly sublimates. As a result, the pressure of the pump channels 182, 183, and 184 increases. Therefore, the fluid (F) filled in the first fluid chamber 173 is moved by the increased pressure away from the gas generating agent 180 toward the second fluid chamber 175 through the main channel 172. Like in the case of the pump unit 150 as shown in FIG. 6, when the gas generating agent 180 is filled in a large area, a laser beam (L2) can be irradiated to the gas generating agent 180 by using a moving energy source from one end to the other end of the gas generating agent 180 as shown by the arrow in FIG. 7.

As described above, according to the present invention, the pump unit does not include a heater, so that a small and integrated fluid treatment device can be provided using the pump unit for applications in the field of microfluidics. Furthermore, the pump unit can be easily applied to a centrifugal microfluidic system having a CD shaped substrate.

In the centrifugal microfluidic system including the pump unit, a fluid can be pumped from the periphery to the center of the CD shaped substrate. Therefore, a microfluidic treatment process including a complex biochemical reaction and a detection process for the reaction can be performed in the CD shaped substrate of the centrifugal microfluidic system, and thus the centrifugal microfluidic system can be easily designed for various microfluidic treatment processes.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A pump unit to move a fluid in a first channel, comprising:
a chamber connected to and fluid communicated to the first channel, the chamber being disposed at a upstream side of the fluid which is to be moved toward a downstream side; and
a gas generating agent contained in the chamber,
wherein the gas generating agent includes a plurality of heating particles; and
wherein the gas generating agent increases air pressure when an energy is applied thereto, thereby moving the fluid toward the downstream direction.

2. The pump unit of claim 1, wherein the gas generating agent further comprises one selected from the group consisting of a sublimation material, azide, and azo compound.

3. The pump unit of claim 2, wherein the sublimation material is one selected from the group consisting of naphthalene, dry ice, iodine, camphor, and paradichlorobenzene.

4. The pump unit of claim 2, wherein the azide is one selected from the group consisting of an inorganic azide, alkyl azide, and aryl azide.

5. The pump unit of claim 2, wherein the azo compound is one selected from the group consisting of AIBN (Azobisisobutyronitrile), ADVN (2,2'-Azobis-(4-methoxy-2,4-dimethylvaleronitrile)), AMBN (2,2'-Azobis-(2-methylbutyronitrile)), ACHN (1,1'-Azobis-(4-cyclohexane-carbonitrile)), ACCN (1,1'-Azobis-(cyclohexanecarbonitrile)), ABAH (2,2'-Azobis-(2-methylbutyronitrile)), and ACVA (1,1'-Azobis-(cyclo-hexanecarbonitrile)).

6. The pump unit of claim 1, further comprising a valve, which is located in the second channel and closes the second channel, wherein the valve opens the second channel when the gas generating agent increases air pressure in the second channel by an application of energy, thereby moving the fluid toward a downstream direction.

7. The pump unit of claim 6, wherein the valve comprises a valve filler filled in at least pant of the second channel, the valve filler including a phase transition material selected from the group consisting of wax, gel, and a thermoplastic resin,
wherein when external energy is supplied to the valve filler, a viscosity of the valve filler reduces, thereby opening the second channel.

8. The pump unit of claim 7, wherein the valve filler further includes a plurality of heating particles dispersed into the phase transition material, the heating particles generating heat by absorbing energy.

9. The pump unit of claim 1, wherein the heating particles are a ferromagnetic material particle or a metal oxide particle.

10. The pump unit of claim 8, wherein the heating particles are a ferromagnetic material particle or a metal oxide particle.

11. The pump unit of claim 2, wherein the sublimation material, azide, and azo compound are present in the form of powder.

12. A centrifugal microfluidic system comprising:
a substrate including a first channel constituting a fluid passage; and
a pump unit moving a fluid in the first channel,
wherein the pump unit comprises:
a chamber formed in the substrate and connected to the first channel, the chamber being disposed at a upstream side of the fluid which is to be moved toward a downstream side; and
a gas generating agent contained in the chamber,
wherein the gas generating agent includes a plurality of heating particles; and
wherein the gas generating agent increases air pressure when an energy is applied thereto, thereby moving the fluid toward the downstream direction.

13. The centrifugal microfluidic system of claim 12, further comprising a driving unit rotating the substrate.

14. The centrifugal microfluidic system of claim 12, which further comprises an external energy source which emits electromagnetic waves towards the gas generating agent.

15. The centrifugal microfluidic system of claim 12, wherein the gas generating agent further comprises one selected from the group consisting of a sublimation material, azide, and azo compound.

16. The centrifugal microfluidic system of claim 15, wherein the sublimation material is one selected from the group consisting of naphthalene, dry ice, iodine, camphor, and paradichlorobenzene.

17. The centrifugal microfluidic system of claim 15, wherein the azide is one selected from the group consisting of an inorganic azide, alkyl azide, and aryl azide.

18. The centrifugal microfluidic system of claim 15, wherein the azo compound is one selected from the group consisting of AIBN (Azobisisobutyronitrile), ADVN (2,2'-Azobis-(4-methoxy-2,4-dimethylvaleronitrile)), AMBN (2,2'-Azobis-(2-methylbutyronitrile)), ACHN (1,1'-Azobis-(4-cyclohexanecarbonitrile)), ACCN (1,1'-Azobis-(cyclohexanecarbonitrile)), ABAH (2,2'-Azobis-(2-methylbutyronitrile)), and ACVA (1,1'-Azobis-(cyclohexanecarbonitrile)).

19. The centrifugal microfluidic system of claim 14, wherein the external energy source emits electromagnetic waves towards the gas generating agent while scanning from an end of the gas generating agent to the other end of the gas generating agent.

20. The centrifugal microfluidic system of claim 12, wherein the chamber of the pump unit is connected to the first channel of the pump unit through a second channel, the pump unit further comprising a valve, which is located in the second channel and closes the second channel; and
wherein the valve opens the second channel when the gas generating agent increases air pressure in the second channel by an application of energy, thereby moving the fluid toward the downstream direction.

21. The centrifugal microfluidic system of claim 20, wherein the valve comprises a valve filler filled in at least part of the second channel, the valve filler including a phase transition material selected from the group consisting of wax, gel and a thermoplastic resin,
wherein when external energy is supplied to the valve filler, a viscosity of the valve filler reduces, thereby opening the second channel.

22. The centrifugal microfluidic system of claim 21, wherein the valve filler further includes a plurality of heating particles dispersed into the phase transition material, the heating particles generating heat by absorbing energy.

23. The centrifugal microfluidic system of claim 12, wherein the heating particles are a ferromagnetic material particle or a metal oxide particle.

24. The centrifugal microfluidic system of claim 22, wherein the heating particles are a ferromagnetic material particle or a metal oxide particle.

25. The centrifugal microfluidic system of claim 15, wherein the sublimation material, azide, and azo compound are present in the form of powder.
